# EUROPÄISCHE PATENTSCHRIFT

(11) **EP 1 511 708 B1**
(45) Veröffentlichungstag und Bekanntmachung des Hinweises auf die Patenterteilung: **28.03.2007**
(21) Anmeldenummer: 03730086.0
(22) Anmeldetag: 22.05.2003
(51) Int. Cl.: C07C 45/62, C07C 49/385

(54) **VERFAHREN ZUR HERSTELLUNG VON CYCLOHEXADECANON**
METHOD FOR PRODUCING CYCLOHEXADECANONE
PROCEDE DE PRODUCTION DE CYCLOHEXADECANONE

(30) Priorität: 29.05.2002 DE 10223915
(43) Veröffentlichungstag der Anmeldung: 09.03.2005
(60) Teilanmeldung: 07101472.4
(73) Patentinhaber: Symrise GmbH & Co. KG, 37603 Holzminden (DE)
(72) Erfinder: WÖHRLE, Ingo, 37603 Holzminden (DE); KUHN, Walter, 37603 Holzminden (DE); FUNK, Hans-Ulrich, 37697 Lauenförde (DE); KÖRBER, Alfred, 37603 Holzminden (DE)
(74) Vertreter: Eisenführ, Speiser & Partner
(86) Internationale Anmeldenummer: PCT/EP2003/005342
(87) Internationale Veröffentlichungsnummer: WO 2003/099753

(56) Entgegenhaltungen:
- EP-A- 1 201 738

## Beschreibung

Die Erfindung betrifft ein Verfahren zur Herstellung von Cyclohexadecanon durch Hydrierung von Cyclohexadecenon und Riechstoffmischungen sowie Produkte enthaltend das erfindungsgemäße Cyclohexadecanon.

Makrocyclische Ketone mit 14 bis 18 gliedrigen Ringen sind allgemein bekannt als Moschusriechstoffe. Cyclohexadecanon ist beispielsweise in Drüsensekreten einiger Zibet-Arten (K. Bauer, A. Garbe, H. Surburg, Common Fragrance and Flavor Materials, S. 169, Wiley-VCH, Weinheim, 1997) enthalten.

Cyclohexadecanon kann gemäß DE-A 2 111 753 durch Hydrierung von 8-Cyclohexadecenon hergestellt werden. In der dort beschriebenen Hydrierung werden sehr hohe Mengen an Hydrierkatalysator eingesetzt.

In EP-A 1201738 wird Cyclohexadecanon durch Hydrierung von 8-Cyclohexadecenon an Palladium in Ethanol hergestellt. Das Gewichtsverhältnis von Palladium zu Cyclohexadecenon lag bei 1:603, die Ausbeute bei etwa 76 % der Theorie.

In Chemistry Letters, 1973, 667-670 wurde aus 5-Cyclohexadecenon mittels Hydrierung Cyclohexadecanon hergestellt. Es sind keine Reaktionsbedingungen angegeben.

Es besteht daher der Bedarf ein Verfahren zu finden, das Cyclohexadecanon wirtschaftlich und in guter parfümistischer Qualität liefert, insbesondere im industriellen Maßstab.

Gegenstand der vorliegenden Erfindung ist ein Verfahren zur Herstellung von Cyclohexadecanon durch Hydrierung von Cyclohexadecenon in Gegenwart von metallischem Palladium, dadurch gekennzeichnet, dass das Gewichtsverhältnis von Palladium zu Cyclohexadecenon unterhalb von 1:5 000 liegt.

Gegenstand der vorliegenden Erfindung sind ebenfalls Lösungen, Riechstoffmischungen und Produkte, bevorzugt parfümierte Produkte, enthaltend das erfindungsgemäß hergestellte Cyclohexadecanon.

Für das erfindungsgemäße Verfahren liegt das Gewichtsverhältnis von Palladium zu Cyclohexadecenon unterhalb von 1:5 000, vorteilhaft im Bereich von 1:8 000 bis 1:5 000 000, bevorzugt im Bereich 1:12 000 bis 1:2 000 000, insbesondere bevorzugt im Bereich 1:20 000 bis 1:800 000, ganz besonders bevorzugt im Bereich von 1:25 000 bis 1:500 000.

Die Palladiummenge bezieht sich dabei auf den absoluten Gehalt des Palladiums, d.h. ohne Trägermaterial und ohne eventuell vorhandenes Wasser oder Verdünnungsmittel.

Cyclohexadecenone im Sinne der Erfindung können ein bis drei olefinische Doppelbindungen enthalten, bevorzugt sind Cyclohexadecenone mit einer olefinischen Doppelbindung.

Erfindungsgemäß können (E)- oder (Z)-Cyclohexadecenone und beliebige Mischungen dieser (E)- oder (Z)-Isomeren eingesetzt werden. Erfindungsgemäß können alle regioisomeren Cyclohexadecenone und beliebige Mischungen dieser Verbindungen eingesetzt werden. Bevorzugt ist die Verwendung von 5-Cyclohexadecenon und 8-Cyclohexadecenon, besonders bevorzugt ist 8-Cyclohexadecenon, wobei ein (E):(Z)-Verhältnis von 1:10 bis 10:1 bevorzugt, ein Verhältnis von 1:5 bis 5:1 besonders bevorzugt und ein Verhältnis von 1:3 bis 4:1 ganz besonders bevorzugt ist. In einer besonders vorteilhaften Ausführungsform wird 8-Cyclohexadecenon mit einem (E):(Z)-Verhältnis von 1:1 bis 3:1 eingesetzt.

Cyclohexadecenone und Verfahren zu ihrer Herstellung sind beispielsweise beschrieben in J. Org. Chem. 1971, 36, 3266-3270; J. Org. Chem. 1971, 36, 4124-4125; Tetrahedron Lett. 1965, 21, 1537-1540 oder auch Bull. Chem. Soc. Jpn. 1980, 53, 2958-2961.

Die in dem erfindungsgemäßen Verfahren eingesetzten Cyclohexadecenone weisen bevorzugt eine geruchlich gute Qualität auf, d.h. dass die verwendeten Cyclohexadecenone chemisch und geruchlich keine wesentlichen störenden Verunreinigungen aufweisen. Die chemische Reinheit liegt bevorzugt bei >96 Gew.%, besonders bevorzugt bei >98 Gew.%.

Das Palladium kann beispielsweise in fein verteilter Form, aufgebracht auf Träger oder zusammen mit anderen Metallen eingesetzt werden (z.B. Mischungen, Legierungen). Die Katalysatoren können Dotierungen mit einem oder mehreren beliebigen Metallen enthalten.

Das Palladium kann auf organische oder anorganische Trägermaterialien aufgebracht sein. Die Katalysatoren können ein Trägermaterial oder Mischungen von Trägermaterialien enthalten. Als vorteilhafte Trägermaterialen seien genannt Aktivkohle, Kohle, Aluminiumoxide, Metalloxide, Kieselgele, Zeolithe, Tone, Tongranulate, amorphe Aluminiumsilicate, oder sonstige anorganische Träger. Ein bevorzugtes Trägermaterial ist Aktivkohle.

Ein ganz besonders bevorzugter Katalysator ist Palladium auf Aktivkohle.

Werden Trägermaterialien enthaltende Katalysatoren verwendet, kann der Anteil an Palladium auf dem Trägermaterial im allgemeinen 0,5 bis 50 Gew.%, bevorzugt 1 bis 20 Gew.%, insbesondere bevorzugt 3 bis 10 Gew.%, ganz besonders bevorzugt 4 bis 7 Gew.% bezogen auf den trockenen Katalysator, betragen.

Für das erfindungsgemäße Verfahren kann der Katalysator im trockenen oder feuchten Zustand (Restfeuchte an Wasser) verwendet werden.

Es ist ebenfalls möglich das Palladium-Metall vor Beginn oder während der Hydrierung in situ durch Reduktion mit Wasserstoff aus entsprechenden Verbindungen wie Oxiden oder Salzen zu erzeugen, wobei gegebenenfalls das Palladium auf einen Träger niedergeschlagen werden kann. Geeignet hierfür sind beispielsweise Palladiumhalogenide wie Palladium-(II)-chlorid.

Das erfindungsgemäße Verfahren kann unter Verwendung von Verdünnungsmitteln oder Verdünnungsmittelgemischen durchgeführt werden. Geeignet sind Verdünnungsmittel, die unter den verwendeten Hydrierbedingungen inert sind, wie beispielsweise ein- oder mehrwertige Alkohole, wässrige Mischungen enthaltend ein- oder mehrwertige Alkohole, Ketone, Ether, Ester, aromatische oder gesättigte Kohlenwasserstoffe. Bevorzugt sind Alkohole mit 1 bis 4 Kohlenstoffatomen, Alkane mit 5 bis 15 Kohlenstoffatomen, Ketone mit 3 bis 8 Kohlenstoffatomen, offenkettige oder cyclische Ether mit 4 bis 10 Kohlenstoffatomen, Ester mit 3 bis 12 Kohlenstoffatomen oder aromatische Kohlenwasserstoffe mit 6 bis 10 Kohlenstoffatomen. Typischerweise können Verdünnungsmittel wie Methanol, Ethanol, Isopropanol, n-Propanol, Isobutanol, n-Butanol, sek.-Butanol, Tetrahydrofuran, Dibutylether, Ethylenglykoldimethylether, Aceton, Butanon, 2-Pentanon, 3-Pentanon, Hexanon, Cyclohexanon, Methylethylketon, Diethylketon, Diisopropylketon, Methylisobutylketon, Ethylacetat, Methylacetat, n-Pentan, n-Hexan, n-Heptan, n-Octan, Isooctan, Cyclopentan, Cyclohexan, Methylcyclohexan, Cyclooctan, Benzol, Toluol, Ethylbenzol, Xylole verwendet werden, bevorzugt sind Ethanol, Isopropanol, Aceton, Methylethylketon und Toluol.

Ebenfalls bevorzugte Verdünnungsmittel sind parfümistisch, dermatologisch oder kosmetisch akzeptable Verdünnungsmittel. Hierbei bevorzugt sind Ethanol, Dipropylenglycol, Propylenglycol, 1,2-Butylenglycol, Glycerin, Diethylenglycolmonoethylether, Diethylphthalat, Isopropylmyristat, Triethylcitrat, Benzylbenzoat und Benzylacetat. Besonders bevorzugt sind Ethanol, Diethylphthalat, Propylenglycol, Dipropylenglycol, Triethylcitrat und Isopropylmyristat.

Das Gewichtsverhältnis von Cyclohexadecenon und Verdünnungsmittel liegt vorteilhafterweise im Bereich von 1:10 bis 4:1, bevorzugt im Bereich von 1:5 bis 3:1, besonders bevorzugt im Bereich von 1:2 bis 2:1. In einer bevorzugten Ausführungsform liegt der Anteil von Cyclohexadecenon bei 40 bis 80 Gew.%, besonders bevorzugt bei 45 bis 75 Gew.%, bezogen auf das Gemisch aus Cyclohexadecenon und Verdünnungsmittel.

Die Hydrierung kann in Gegenwart von Verdünnungsmitteln bei Temperaturen von 0 bis 150°C durchgeführt werden. Vorteilhaft sind Temperaturen im Bereich von 10 bis 100°C, bevorzugt im Bereich von 20 bis 85°C, insbesondere bevorzugt bei 30 bis 60°C.

Die Hydrierung kann auch in Abwesenheit von Verdünnungsmitteln bei Temperaturen oberhalb von 60°C durchgeführt werden, bevorzugt im Bereich von 70 bis 150°C, insbesondere bevorzugt bei 70 bis 100°C. Eine hohe und/oder zu lange thermische Belastung ist der geruchlichen Qualität des Cyclohexadecanons abträglich. Nach der Hydrierung in Abwesenheit von Verdünnungsmitteln wird bevorzugt ein Reinigungsschritt durchgeführt, der bevorzugt eine Umkristallisation umfasst.

Erfindungsgemäß werden die Hydrierungen mit elementarem Wasserstoff durchgeführt.

Der Wasserstoffdruck liegt geeigneterweise bei 1 bis 100 bar, bevorzugt bei 1 bis 30 bar, insbesondere bevorzugt bei 3 bis 20 bar und ganz besonders bevorzugt bei 5 bis 15 bar.

Die Reaktionszeit der Hydrierung beträgt bevorzugt 2 bis 80 Stunden, insbesondere bevorzugt 5 bis 40 Stunden, ganz besonders bevorzugt 8 bis 25 Stunden.

Nach der Hydrierung kann eine Umkristallisation nach Abtrennung des Katalysators von Vorteil sein, insbesondere in Bezug auf die geruchliche Qualität.

Besonders vorteilhaft ist die Umkristallisation aus Alkoholen mit 1 bis 8 Kohlenstoffatomen, Alkanen mit 5 bis 15 Kohlenstoffatomen, Ketonen mit 3 bis 8 Kohlenstoffatomen, offenkettigen oder cyclischen Ethern mit 4 bis 10 Kohlenstoffatomen, Estern mit 3 bis 12 Kohlenstoffatomen oder aromatischen Kohlenwasserstoffen mit 6 bis 10 Kohlenstoffatomen. Bevorzugt sind Methanol, Ethanol, Isopropanol, n-Propanol, Isobutanol, n-Butanol, sek.-Butanol, Tetrahydrofuran, Dibutylether, Ethylenglykoldimethylether, Aceton, Butanon, 2-Pentanon, 3-Pentanon, Hexanon, Cyclohexanon, Methylisobutylketon, Ethylacetat, Methylacetat, n-Pentan, n-Hexan, n-Heptan, n-Octan, Isooctan, Cyclopentan, Cyclohexan, Methylcyclohexan, Cyclooctan, Benzol, Toluol, Ethylbenzol, Xylole, besonders bevorzugt sind Methanol, Ethanol, Aceton, Isopropanol und Toluol.

In einer besonders vorteilhaften Ausführungsform wird die thermische Belastung während des gesamten Verfahrens, d.h. der Hydrierung und der sich gegebenenfalls anschließenden Reinigung (z.B. Kristallisation oder Destillation) bei Temperaturen kleiner oder gleich 150°C, bevorzugt bei Temperaturen kleiner oder gleich 130°C und besonders bevorzugt bei Temperaturen kleiner oder gleich 100°C durchgeführt.

Das erfindungsgemäß hergestellte Cyclohexadecanon ist eine sehr komplexe Muskverbindung und weist ein wertvolles geruchliches Spektrum auf Bei der parfümistischen Beschreibung zeigt Cyclohexadecanon große Ähnlichkeiten zu Muscon und Cyclopentadecanon sowie eine starke Makromusk Note, darüber hinaus zeichnet sich Cyclohexadecanon durch die betont animalische Note von Moschus-Tinktur oder natürlichem Tonkinmoschus aus. Weitere geruchlich interessante Aspekte sind Ambrette und Nitromoschus, daneben im Angeruch eine gewisse Fruchtigkeit.

Das erfindungsgemäß hergestellte Cyclohexadecanon besitzt schon als Hydrierungsrohprodukt, insbesondere bei Durchführung der Hydrierung in Gegenwart von Verdünnungsmitteln, eine gute parfümistische Qualität. Unter dem Hydrierungsrohprodukt wird das direkt aus der Hydrierung stammende Produkt nach Entfernen des Hydrierkatalysators verstanden. Das Hydrierungsrohprodukt kann z.B. als unmittelbare Lösung in dem bei der Hydrierung verwendeten Verdünnungsmittel vorliegen, durch partielles Entfernen des Verdünnungsmittels in diesem angereichert werden oder nach Entfernen des Verdünnungsmittels als reine Substanz erhalten werden.

Wenn das Hydrierungsrohprodukt als Lösung verwendet oder weiterverarbeitet werden soll, so ist es vorteilhaft, die Hydrierung in Gegenwart eines parfümistisch, dermatologisch oder kosmetisch akzeptablen Verdünnungsmittels durchzuführen, damit die das Hydrierungsrohprodukt enthaltende Lösung als solche verwendet werden kann. Das erfindungsgemäße Hydrierungsrohprodukt kann, insbesondere als Lösung in dem Verdünnungsmittel, in dessen Gegenwart die Hydrierung durchgeführt wurde, ohne weitere Aufreinigungsschritte als Riechstoff, in parfümistischen Kompositionen und zur Beduftung verwendet werden.

Das erfindungsgemäße Verfahren kann z.B. wie folgt durchgeführt werden:

In einem Druckbehälter mit Rührer werden Cyclohexadecenon und der Katalysator und gegebenenfalls ein Verdünnungsmittel vorgelegt. Es wird bei der gewählten Reaktionstemperatur und Wasserstoffdruck hydriert. Das Hydrierungsrohprodukt wird typischerweise nach Entfernen des Katalysators durch Filtration, Dekantieren oder Zentrifugation erhalten. Bei einer verdünnungsmittelfreien Hydrierung muss die Abtrennung des Katalysators bei höherer Temperatur erfolgen, die vorteilhafterweise oberhalb der Schmelztemperatur des Cyclohexadecanons liegt. Gegebenenfalls kann eine weitergehende Aufreinigung durchgeführt werden, beispielsweise durch Destillation, Kristallisation oder Wasserdampfbehandlung. Nach dem erfindungsgemäßen Verfahren kann das Cyclohexadecanon in nahezu quantitativer Ausbeute erhalten werden (meist größer als 97 Gew.%).

Nach dem erfindungsgemäßen Verfahren kann Cyclohexadecanon in hoher Reinheit und in sehr guter Ausbeute hergestellt werden. Dadurch ist das erfindungsgemäße Verfahren besonders in wirtschaftlicher, technischer und industrieller Hinsicht vorteilhaft.

Das nach dem erfindungsgemäßen Verfahren hergestellte Cyclohexadecanon kann insbesondere verwendet werden als Riechstoff, in Parfümkompositionen, Parfümölen, Riechstoffinischungen oder Duftkompositionen.

Riechstoffe, mit denen das Cyclohexadecanon kombiniert werden kann sind beispielsweise beschrieben in Bauer, Garbe, Surburg, Common Fragrance and Flavor Materials, Wiley-VCH, 4. Aufl., 2001.

Ein weiteres Anwendungsgebiet des Cylohexadecanons bzw. der das Cylohexadecanon enthaltenen Riechstoffinischungen sind parfümierte Produkte, bevorzugt Hygiene- oder Pflegeprodukte, insbesondere im Bereich des Haushaltes und der Körperpflege.

Cyclohexadecanon zeichnet sich besonders durch eine gute Haftung auf synthetischen und natürlichen Fasern, Haaren sowie der Haut aus.

Die das erfindungsgemäß hergestellte Cyclohexadecanon enthaltenden Parfümöle können in konzentrierter Form, in Lösungen oder in sonstiger modifizierter Form verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchem sowie die Parfümierung von sauren, alkalischen und neutralen Reinigungsmitteln, wie z.B. Fußbodenreinigem, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes- und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes oder Produkten der dekorativen Kosmetik.

Folgende Beispiele erläutern die Erfindung.

### Beispiel 1

In Beispiel 1 liegt das Verhältnis von Pd:8-Cyclohexadecenon bei etwa 1:18 520.

In einem Rührautoklaven mit Begasungsrührer wurden 100 kg 8-Cyclohexadecenon (geruchlich gute Qualität / das gaschromatographische (GC) Verhältnis von (E)- und (Z)-Isomeren lag bei 3:1) und 180 g Palladium auf Aktivkohle (Pd-Gehalt: 5 Gew. %) mit 40 % Wassergehalt vorgelegt. Es wurde 17 Stunden bei 75°C und 20 bar Wasserstoffdruck hydriert und anschließend bei etwa 75°C filtriert. Die Ausbeute an Hydrierungsrohprodukt lag bei >98 % (GC-Gehalt an Cyclohexadecanon: >97 %). Durch Umkristallisation aus Methanol oder durch Wasserdampf behandlung konnte die parfümistische Qualität noch weiter gesteigert werden.

### Beispiel 2

In Beispiel 2 liegt das Verhältnis von Pd:8-Cyclohexadecenon bei etwa 1:7 500.

In einem Rührautoklaven mit Begasungsrührer wurden 180 kg 8-Cyclohexadecenon (geruchlich gute Qualität / GC-Gehalt an (E)- und (Z)-Isomeren: 65:34) in 180 kg Diethylphthalat und 800 g Palladium auf Aktivkohle (Pd-Gehalt: 5 Gew. %) mit 40 % Wassergehalt vorgelegt. Es wurde 20 Stunden bei 49 bis 52°C und 5 bar Wasserstoffdruck hydriert. Nach Filtration wurden 349 kg Cyclohexadecanon als 50 %ige Lösung in Diethylphthalat erhalten. Die umsatzbezogene Ausbeute an Cyclohexadecanon lag bei >99 %. Die so erhaltene Ware hatte eine gute parfümistische Qualität.

### Beispiel 3

In Beispiel 3 liegt das Verhältnis von Pd:8-Cyclohexadecenon bei etwa 1:15 000.

In einem Rührautoklaven mit Begasungsrührer wurden 180 kg 8-Cyclohexadecenon (geruchlich gute Qualität / GC-Gehalt an (E)- und (Z)-Isomeren: 64:34,5) in 180 kg Isopropylmyristat und 400 g Palladium auf Aktivkohle (Pd-Gehalt: 5 Gew. %) mit 40 % Wassergehalt vorgelegt. Es wurde 40 Stunden bei 4 bis 5 bar Wasserstoffdruck hydriert, wobei die Temperatur von anfangs 50°C im Laufe der Hydrierung auf 125°C erhöht wurde. Nach Filtration wurden 346 kg Cyclohexadecanon als 50 %ige Lösung in Isopropylmyristat erhalten. Die umsatzbezogene Ausbeute an Cyclohexadecanon lag bei >99 %. Die so erhaltene Ware hatte eine gute parfümistische Qualität.

### Beispiel 4

In Beispiel 4 liegt das Verhältnis von Pd:8-Cyclohexadecenon bei etwa 1:33 333.

In einem Rührautoklaven mit Begasungsrührer wurden 100 kg 8-Cyclohexadecenon (geruchlich gute Qualität / das GC-Verhältnis von (E)- und (Z)-Isomeren lag bei 2,5:1), 45 kg Aceton und 100 g Palladium auf Aktivkohle (Pd-Gehalt: 5 Gew. %) mit 40 % Wassergehalt vorgelegt. Es wurde 12 Stunden bei 35 bis 40°C und 15 bar Wasserstoffdruck hydriert und anschließend filtriert. Nach Entfernen des Acetons wurden 99,2 kg Cyclohexadecanon erhalten (GC-Gehalt an Cyclohexadecanon: >98 %). Die so erhaltene Ware hatte eine gute parfümistische Qualität. Durch Umkristallisation aus Methanol konnte die parfümistische Qualität noch weiter gesteigert werden.

### Beispiel 5

In Beispiel 5 liegt das Verhältnis von Pd : 8-Cyclohexadecenon bei etwa 1 : 5 556.

In einem Rührautoklaven mit Begasungsrührer wurden 1000 kg 8-Cyclohexadecenon (geruchlich gute Qualität / das GC-Verhältnis der (E)- und (Z)-Isomere lag bei 2:1) in 1000 kg Ethanol und 6 kg Palladium auf Aktivkohle (Pd-Gehalt: 5 Gew. %) mit 40 % Wassergehalt vorgelegt. Es wurde 18 Stunden bei 30 bis 50°C und 6 bar Wasserstoffdruck hydriert. Nach Filtration wurden 2002 kg einer 50 %igen Lösung von Cyclohexadecanon in Ethanol (GC-Reinheit ohne Ethanol >97 %) erhalten. Die Ausbeute an Cyclohexadecanon lag bei >99 %. Die so erhaltene Ware hatte eine gute parfümistische Qualität. Durch Umkristallisation aus Ethanol konnte die parfümistische Qualität noch weiter gesteigert werden.

### Beispiel 6

In Beispiel 6 liegt das Verhältnis von Pd:8-Cyclohexadecenon bei etwa 1:7 778.

In einem Rührautoklaven mit Begasungsrührer wurden 700 g 8-Cyclohexadecenon (geruchlich gute Qualität / das GC-Verhältnis von (E)- und (Z)-Isomeren lag bei 63,4:35,5), 300 g Aceton und 3 g Palladium auf Aktivkohle (Pd-Gehalt: 5 Gew. %) mit 40 % Wassergehalt vorgelegt. Es wurde 8 Stunden bei 55-60°C und 5 bar Wasserstoffdruck hydriert und anschließend filtriert. Es wurden 992 g Hydrierungsrohprodukt erhalten, die GC-Reinheit des Cyclohexadecanons lag bei 99,4 % (Aceton nicht berücksichtigt). Die so erhaltene Ware hatte eine gute parfümistische Qualität.

### Beispiel 7

In Beispiel 7 liegt das Verhältnis von Pd:8-Cyclohexadecenon bei etwa 1:6 667.

In einem Rührautoklaven mit Begasungsrührer wurden 800 g 8-Cyclohexadecenon (geruchlich gute Qualität / das Verhältnis von (E)- und (Z)-Isomeren lag bei 1,8:1), 400 g Aceton und 4 g Palladium auf Aktivkohle (Pd-Gehalt: 5 Gew. %) mit 40 % Wassergehalt vorgelegt. Es wurde 8 Stunden bei 25°C und 18 bar Wasserstoffdruck hydriert und anschließend filtriert. Es wurden 991 g Hydrierungsrohprodukt erhalten, die GC-Reinheit des Cyclohexadecanons lag bei 99,3 % (Aceton nicht berücksichtigt). Die so erhaltene Ware hatte eine gute parfümistische Qualität.

### Beispiel 8

In Beispiel 8 liegt das Verhältnis von Pd:8-Cyclohexadecenon bei etwa 1:8 317.

In einem Rührautoklaven mit Begasungsrührer wurden 998 g 8-Cyclohexadecenon (geruchlich gute Qualität) (der GC-Gehalt von (E)- und (Z)-Isomeren lag bei 63,4:35,5) und 4 g Palladium auf Aktivkohle (Pd-Gehalt: 5 Gew. %) mit 40 % Wassergehalt vorgelegt. Es wurde 15 Stunden bei 95°C und 18 bar Wasserstoffdruck hydriert und anschließend bei etwa 95°C filtriert. Die Ausbeute an Hydrierungsrohprodukt lag bei >98 % (GC-Gehalt an Cyclohexadecanon betrug 96 %). Durch Umkristallisation aus Methanol oder durch Wasserdampfbehandlung konnte die parfümistische Qualität noch weiter gesteigert werden.

## Patentansprüche

1. Verfahren zur Herstellung von Cyclohexadecanon durch Hydrierung von Cyclohexadecenon in Gegenwart von metallischem Palladium, **dadurch gekennzeichnet, dass** das Gewichtsverhältnis von Palladium zu Cyclohexadecenon unterhalb von 1:5 000 liegt.

2. Verfahren nach Anspruch 1, **dadurch gekennzeichnet, dass** das Gewichtsverhältnis von Palladium zu Cyclohexadecenon im Bereich von 1:8 000 bis 1:5 000 000 liegt.

3. Verfahren nach Anspruch 1 oder 2, **dadurch gekennzeichnet, dass** als Hydrierkatalysator Palladium auf Aktivkohle verwendet wird.

4. Verfahren nach wenigstens einem der Ansprüche 1 bis 3, **dadurch gekennzeichnet, dass** 8-Cyclohexadecenon eingesetzt wird.

5. Verfahren nach wenigstens einem der Ansprüche 1 bis 4, **dadurch gekennzeichnet, dass** die Hydrierung in Gegenwart eines Verdünnungsmittels durchgeführt wird und das Gewichtsverhältnis von Cyclohexadecenon und Verdünnungsmittel im Bereich von 1:10 bis 4:1 liegt.

6. Verfahren nach wenigstens einem der Ansprüche 1 bis 5, **dadurch gekennzeichnet, dass** die Hydrierung in Abwesenheit eines Verdünnungsmittels bei Temperaturen im Bereich 70 bis 150°C durchgeführt wird.

7. Verfahren nach wenigstens einem der Ansprüche 1 bis 6, **dadurch gekennzeichnet, dass** die Hydrierung in einem parfümistisch, dermatologisch oder kosmetisch akzeptablen Verdünnungsmittel durchgeführt wird.

8. Verfahren nach wenigstens einem der Ansprüche 1 bis 7, **dadurch gekennzeichnet, dass** das Verdünnungsmittel gewählt ist aus der Gruppe Ethanol, Isopropanol, Aceton, Methylethylketon, Toluol, Diethylphthalat, Propylenglycol, Dipropylenglycol, Triethylcitrat und Isopropylmyristat.

9. Verfahren nach wenigstens einem der Ansprüche 1 bis 8, **dadurch gekennzeichnet, dass** nach der Hydrierung eine Umkristallisation durchgeführt wird.

10. Verfahren nach wenigstens einem der Ansprüche 1 bis 9, **dadurch gekennzeichnet, dass** die Reaktionszeit der Hydrierung 2 bis 80 Stunden beträgt.

11. Lösung enthaltend ein nach einem der Ansprüche 1 bis 10 hergestelltes Cyclohexadecanon sowie ein Verdünnungsmittel ausgewählt aus der Gruppe bestehend aus Dipropylenglycol, Propylenglycol, 1,2-Butylenglycol, Glycerin, Diethylenglyrolmonoethylether, Isopropylmyristat, Triethylcitrat, Benzylbenzoat und Benzylacetat.

## Claims

1. Process for the preparation of cyclohexadecanone by hydrogenation of cyclohexadecenone in the presence of palladium metal, **characterised in that** the weight ratio of palladium to cyclohexadecenone is less than 1:5000.

2. Process according to claim 1, **characterised in that** the weight ratio of palladium to cyclohexadecenone is in the range from 1:8000 to 1:5,000,000.

3. Process according to claim 1 or 2, **characterised in that** palladium on activated carbon is used as the hydrogenation catalyst.

4. Process according to at least one of claims 1 to 3, **characterised in that** 8-cyclohexadecenone is used.

5. Process according to at least one of claims 1 to 4, **characterised in that** the hydrogenation is carried out in the presence of a diluent and the weight ratio of cyclohexadecenone to diluent is in the range from 1:10 to 4:1.

6. Process according to at least one of claims 1 to 5, **characterised in that** the hydrogenation is carried out in the absence of a diluent at temperatures in the range from 70 to 150°C.

7. Process according to at least one of claims 1 to 6, **characterised in that** the hydrogenation is carried out in a perfumistically, dermatologically or cosmetically acceptable diluent.

8. Process according to at least one of claims 1 to 7, **characterised in that** the diluent is selected from the group ethanol, isopropanol, acetone, methyl ethyl ketone, toluene, diethyl phthalate, propylene glycol, dipropylene glycol, triethyl citrate and isopropyl myristate.

9. Process according to at least one of claims 1 to 8, **characterised in that** a recrystallisation is carried out after the hydrogenation.

10. Process according to at least one of claims 1 to 9, **characterised in that** the reaction time of the hydrogenation is from 2 to 80 hours.

11. Solution comprising a cyclohexadecanone prepared according to any one of claims 1 to 10 and a diluent selected from the group consisting of dipropylene glycol, propylene glycol, 1,2-butylene glycol, glycerol, diethylene glycol monoethyl ether, isopropyl myristate, triethyl citrate, benzyl benzoate and benzyl acetate.

## Revendications

1. Procédé pour la préparation de la cyclohexadécanone par hydrogénation de la cyclohexadécénone en présence de palladium métallique, **caractérisé en ce que** les proportions relatives en poids entre le palladium et la cyclohexadécénone sont inférieures à 1:5000.

2. Procédé selon la revendication 1, **caractérisé en ce que** les proportions relatives en poids entre le palladium et la cyclohexadécénone se situent dans l'intervalle de 1:8000 à 1:5 000 000.

3. Procédé selon la revendication 1 ou 2, **caractérisé en ce que** le catalyseur utilisé pour l'hydrogénation est le palladium sur charbon actif.

4. Procédé selon au moins une des revendications 1 à 3, **caractérisé en ce que** l'on met en oeuvre la 8-cyclohexadécénone.

5. Procédé selon au moins une des revendications 1 à 4, **caractérisé en ce que** l'hydrogénation est réalisée en présence d'un diluant et **en ce que** les proportions relatives en poids entre la cyclohexadécénone et le diluant se situent dans l'intervalle de 1:10 à 4:1.

6. Procédé selon au moins une des revendications 1 à 5, **caractérisé en ce que** l'hydrogénation est réalisée en l'absence d'un diluant, à des températures dans l'intervalle de 70 à 150°C.

7. Procédé selon au moins une des revendications 1 à 6, **caractérisé en ce que** l'hydrogénation est réalisée dans un diluant acceptable pour des applications parfumistiques, dermatologiques ou cosmétiques.

8. Procédé selon au moins une des revendications 1 à 7, **caractérisé en ce que** le diluant est choisi dans le groupe consistant en l'éthanol, l'isopropanol, l'acétone, la méthyléthylcétone, le toluène, le phtalate de diéthyle, le propylèneglycol, le dipropylèneglycol, le citrate de triéthyle et le myristate d'isopropyle.

9. Procédé selon au moins une des revendications 1à 8, **caractérisé en ce que**, après l'hydrogénation, on procède à une recristallisation.

10. Procédé selon au moins une des revendications 1 à 9, **caractérisé en ce que** la durée de la réaction d'hydrogénation est de 2 à 80 h.

11. Solution contenant une cyclohexadécanone préparée selon l'une des revendications 1 à 10 et un diluant choisi dans le groupe consistant en le dipropylèneglycol, le propylèneglycol, le 1,2-butylèneglycol, le glycérol, l'éther monoéthylique du diéthylèneglycol, le myristate d'isopropyle, le citrate de triéthyle, le benzoate de benzyle et l'acétate de benzyle.
